# EUROPEAN PATENT APPLICATION

(11) **EP 2 949 270 A1**
(43) Date of publication of application: **02.12.2015**
(21) Application number: 15169629.1
(22) Date of filing: 28.05.2015
(51) Int. Cl.: A61B 5/103, A43D 1/02

(54) **STRUCTURAL ARRANGEMENT FOR AN APPARATUS FOR EXAMINATION OF THE PRESSURE LOAD OF THE SOLE**

(30) Priority: 28.05.2014 HU 1400119 U
(71) Applicant: Podiart Kft., 1184 Budapest (HU)
(72) Inventor: Imre, Kökényesi, 2367 Újhartyán (HU)
(74) Representative: Harangozo, Gabor

(57) **Abstract**

Structural arrangement for an apparatus for the examination of the pressure load of the sole, comprising an examination plate made preferably of glass for supporting one sole or both soles of an examined person; a pair of mirrors (7) arranged in longitudinal direction between soles of the examined person and used as an optical element for projecting an image of an inner ankle region of the feet (4) of the examined person on a further mirror (8) constituting an optical element and arranged under and parallel to the examination plate (2), one or more image recording devices arranged in a plane between the soles of the examined person and being in optical connection with said mirror (8), wherein one of the image recording devices and a further optical element is arranged along the longitudinal axis of the soles of the examined person and assigned to the heel region of the examined sole for combining an image of the heel and at least a partial region of a leg of the examined person with an image reflected by the mirror (8) or delivered by the camera (9).

## Description

The invention relates to a structural arrangement for an apparatus for examination of pressure load of soles comprising an examination plate made of at least translucent material, for supporting the sole or both soles of the examined person; at least one image recording device being in visual connection with the sole or both soles of the examined person; and at least one optical element inserted in an optical path between the examination plate and at least one image recording device.

Most diseases of the foot are caused by static foot deformities. The static components constituting the foot - bones, tendons, joints, muscles - are brought into an abnormal position or functional disorders occur. Since the foot is responsible not only for supporting, but also for moving the body, static disorders are then conducive to minor or major mobility impairments.

Static foot problems are treated through the correction of the static superstructure. As for the method of correction, that means primarily and most often the correction of the supports, i.e. healing by an insole. In order to make an adequate insole, the static position of the foot needs to be known. Where a pathological static condition is identified, that needs to be corrected appropriately. Correction is administered with the help of a specific insole assembled of modular components. This, in turn, requires the determination of the exact position and size of the correction elements, in other words, the modules.

Various methods and apparatuses are known for conducting the orthopaedic examinations to determine the disorders and deformities of the sole; these serve also as the basis for making adequate therapeutic insoles.

The apparatuses concerned record a so-called pressure point image of the sole that can be regarded as a kind of isobar. The distribution of the areas subject to different pressure loads can be visualised by various computational techniques, and the characteristics of the resulting image will correlate with the known static foot diseases. Several methods are known for recording pressure distribution images. The essence of one of the most frequently used examination methods is that the patient stands barefoot on a glass sheet with proper lighting, installed in a closed box, and the light beam reflected from the other side of the glass sheet is processed.

Such a structural arrangement is disclosed for example in the utility model description No. HU-4255, referred to and relied on in the present application. This structural arrangement comprises a closed box or enclosure with two glass sheets as its cover plates, and each of the glass sheets is lit by a linear light source. The structural arrangement itself has a camera that transmits the image taken of the feet on the glass sheets through the camera, and then it processes and creates the pressure distribution image. During the use of the structural arrangement, the patient stands on the transparent glass sheet. The photometric phenomenon, described in detail in HU-4255 occurs when the skin of the sole comes in contact with the glass sheet; at the same time, light passes through the glass also without refraction and, under the effect of the light-reflecting medium (the glass sheet) put in its way, it behaves according to the optical laws. The camera in the box detects the light through an appropriate mirror arrangement. The camera is connected to a computer that processes the transmitted signals corresponding to the pressure point image of the sole, and displays the pressure distribution image in the known way. That is, the processing of the measuring signal, signal transmission included, is well-known in itself. Several apparatuses based on a similar principle exist for preparing the pressure distribution, i.e. the pressure map, of the sole.

The pressure distribution image transmitted by the camera is processed by computing means assigning specific colours to the fields characterised by different degrees of brightness. The resulting image is pretty graphical and suitable for diagnosing specific diseases, and it is also adequate for making insoles.

This solution is basically reliable and offers good results, but it does not provide for the concurrent acquisition of every piece of information that may be needed, and its use is relatively time-consuming, which makes its use on a mass scale rather disadvantageous.

The underlying principle of the proposed solution is to innovatively upgrade and supplement the known arrangement so as to obtain all the necessary information and data, in particular the angle enclosed by the leg and the heel, in one examination that is quick and relatively simple.

Said task has been solved by a structural arrangement for an apparatus for the examination of the pressure load of the sole, comprising an examination plate made of at least translucent material, for supporting the sole or both soles of the examined person; at least one image recording device being in visual connection with the sole or both soles of the examined person; and at least one optical element inserted in an optical path between the examination plate and at least one image recording device. In a novel way the arrangement further comprises a V-shaped pair of mirrors arranged in longitudinal direction between the two soles of the examined person and used as an optical element for projecting an image of an inner ankle region of the feet of the examined person onto a further mirror constituting an optical element and arranged under and parallel to the examination plate. The at least one image recording device comprises a camera arranged in a plane between the soles of the examined person and being in optical connection with said mirror, and one of the image recording device and a further optical element is arranged along the longitudinal axis of the soles of the examined person and assigned to the heel region of the examined sole for combining an image of the heel and at least a partial region of a leg of the examined person with an image reflected by the mirror or delivered by the camera.

According to a preferred embodiment of the structural arrangement according to the invention that the examination plate is made of glass.

According to a further preferred embodiment of the structural arrangement according to the invention the image recording device comprises a camera.

According to a further preferred embodiment of the structural arrangement according to the invention the optical element comprises a mirror.

According to a further preferred embodiment of the structural arrangement according to the invention the examination plate comprises a transparent plate.

According to a further preferred embodiment of the structural arrangement according to the invention a further mirror is arranged over the examination plate and tilted 45 degrees away from the plane of the examination plate, along the longitudinal axis of the soles of the examined person.

According to a further preferred embodiment of the structural arrangement according to the invention a further camera is arranged under the examination plate along the longitudinal axis of the soles of the examined person.

According to a further preferred embodiment of the structural arrangement according to the invention said camera is arranged spaced from and behind the heel region of the examined sole and directed to the sole and at least a partial region of a leg of the examined person.

In a further preferred embodiment the structural arrangement according to the invention comprises plane mirrors.

In a further preferred embodiment the structural arrangement according to the invention comprises at least one internal light source arranged in a manner projecting the emitted light via the optical elements onto one or both examined soles.

In a further preferred embodiment and in case of simultaneous examination of both soles the arrangement comprises a single further image recording device and further optical elements assigned to the soles and arranged between them, wherein each of the further optical elements is arranged in longitudinal direction between the soles and tilted away from the vertical plane in opposite directions.

In a further preferred embodiment and in case of simultaneous examination of both soles the structural arrangement according to the invention comprises a further image recording device and a further optical element assigned to each of the soles.

In a further preferred embodiment the structural arrangement according to the invention comprises a mechanical stiffening inlay associated with the examination plate.

According to a further preferred embodiment of the structural arrangement according to the invention the arrangement is built in combination with a means for measuring the body weight and/or body fat percentage.

One advantage of the structural arrangement is that it provides more, and more precise, visual information on the foot and the sole due to its innovative mirror and camera arrangement. Concurrent images are obtained of the sagittal axis of both feet; the angle enclosed by the axes of the leg and the heel, respectively. In possession of this information, we can correct the inclination of the heel. A picture is obtained of the internal surface of the feet, the height of the longitudinal arch. In possession of this information, we can choose the module needed for the correction of the longitudinal arch.

With only three cameras, a flat mirror and a "V" mirror, this innovative arrangement can concurrently record the ensemble of images needed for making the exact correction. With this method, we can define exactly the leg/heel inclination angle, the basis for modern insole-making. The structural arrangement is, moreover, capable of taking a pressure-point shot of the sole in the known way, so the pressure distribution of the sole, necessary for the appropriate placement of the transverse arch wedge, becomes visible. Information obtained can be stored in digital format in any way known to those skilled in the art, thus for example graphic utilities can be made for the correct interpretation of the pictures. Any distorted shots can be rendered undistorted by using a computer programme.

The invention will be described in more detail on the basis of the attached drawings showing exemplary implementations of the proposed solution. In the drawings,
- Figure 1A-1C: show the relative position of the leg and heel bone axes;
- Figure 2: is a schematic view of the size of the internal longitudinal arch of the foot;
- Figure 3: is a schematic view of an arrangement suitable for recording the image of the sole;
- Figure 4: outlines a device suitable for recording the internal region of the leg around the inner ankle;
- Figure 5: is a schematic view of a device for recording the image of the leg and the heel;
- Figure 6: is a schematic view of a further possible arrangement for recording the image of the leg and the heel;
- Figure 7: shows a proposed arrangement in plan view, and
- Figure 8: is a perspective view of the arrangement according to Figure 7.

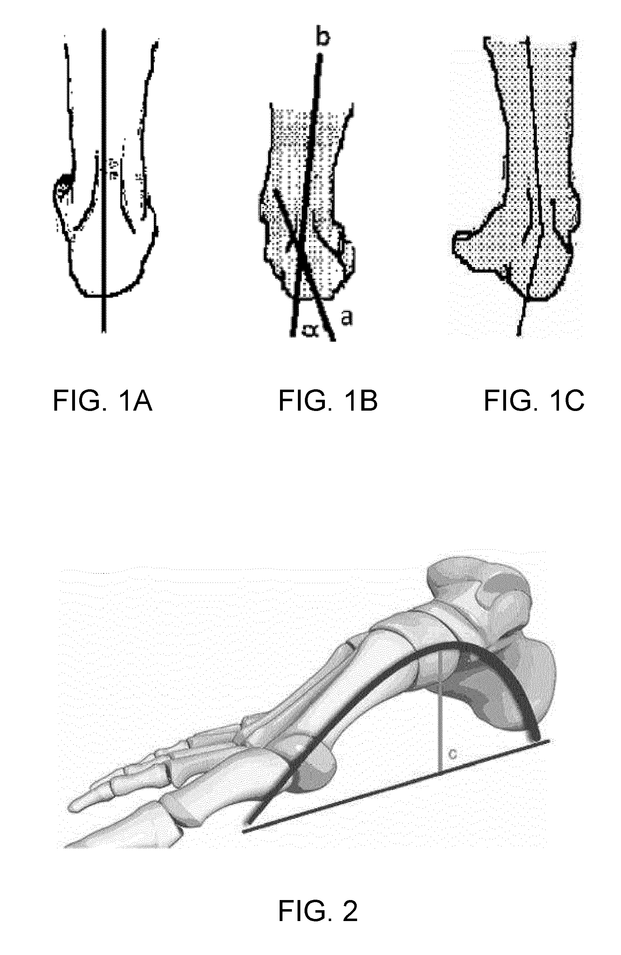

The lower limb of the human body is a sophisticated locomotion apparatus, comprising the joint comprising the leg and the foot (the talocrural joint) as distinctive point. The leg and the astragalus fit casting-like into this joint from above and from below, respectively. The proper (horizontal) status of this joint is important for the correct functioning of the leg. The astragalus, in a hidden position, invisible from the outside, is located between the heel bone and the tibia. One can infer its position from the examination of the heel bone that is tightly linked to it. If the axes of the leg and the heel, respectively, are not parallel as in the healthy condition outlined in Figure 1A, this is a pathological condition (valgus deformity in Figure 1B or varus deformity in Figure 1C). Making these two, **a** and **b,** axes parallel are the first and foremost task during the correction of the foot.

Axis **b** of the tibia, axis **a** of the heel bone and angle **α** enclosed by the two can be determined on the basis of a shot taken from the rear camera angle applied in the structural arrangement presented above. It is easy to understand that, in order to make the two axes **a** and **b** parallel, a wedge having exactly the rising gradient of **α** needs to be placed under the heel concerned.

It is easy to understand on the basis of Figure 1B that angle **α** enclosed by the two, **a, b,** axes is identical with the rising gradient of the correction module, that is, the leg/heel axes **a** and **b** will be made parallel by placing a corresponding module under the sole, and this will make the articulatio talocruralis consisting of the astragalus and the tibia parallel as well, that is, the joint is corrected.

In real life, the correction is realised most generally by using the appropriate member of a series of heel cushions, i.e. a rubber module with a rising gradient corresponding to axis deviation angle **α**.

The foot has an arched structure. One consequence of flatfoot is the reduction of height **c** of the longitudinal arch as outlined in Figure 2 but, in reality, the abnormally high arch that may be a disease of its own or the concomitant of some other static problem, is a more frequent disorder. Therefore, measuring height **c** of the longitudinal arch is a similarly essential task.

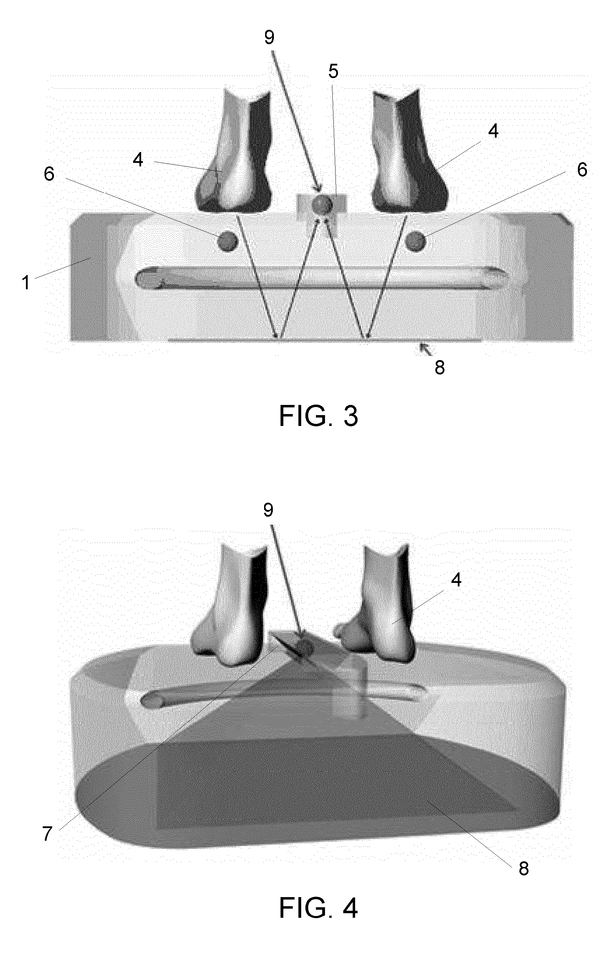

A possible embodiment of the proposed structural arrangement and the layout of its essential parts is outlined in the further drawings. Figure 3 shows only one possible and preferred exemplary embodiment of the structural arrangement according to the invention. Figure 3 shows a possible layout of mirrors and cameras used as optical elements and image-recording devices in perspective view, and Figure 4 shows them in plan view. A podoscope 1 of any design whatsoever ensuring functionality, comprises as usual dedicated surfaces 3 on its examination plate 2 made of glass, upon which a foot or feet 4 or more precisely one or both soles to be examined is/are be placed. The feet 4 are separated by a rib 5. A rear camera 6 used as an image-recording device is arranged in each of the longitudinal median planes of the surfaces 3, behind said surfaces 3 in the proximity of the rear wall of the podoscope 1. An intermediate V-shaped pair of mirrors 7 serving as an optical element is located on the two longitudinal sides of rib 5, whereas under or under each of the surfaces 3, a bottom mirror 8 is arranged. There is a further intermediate camera 9 arranged in the rib 5, in its medium range.

As can be seen in Figure 3, the image of the sole of foot 4 placed on the examination plate 2 of the podoscope 1 is reflected by the mirror 8 fitted in the podoscope 1 to the intermediate camera 9 installed in the rib 5 and facing vertically downwards. The image of the sole is recorded in that way.

To take a shot of the internal region around the inner ankle of the foot 4, the V-shaped pair of mirrors 7 that is protected by the rib 5 needs to be positioned between the feet 4. This ensures that the mirrors inclined at an angle of 45° of the pair of mirrors 7 reflect the inner image of the feet 4 onto the same mirror 8 as the image of the sole, that is, with the presented arrangement, the two inner ankles will appear in between the images of the soles. This is shown in the schematic representation in Figure 4.

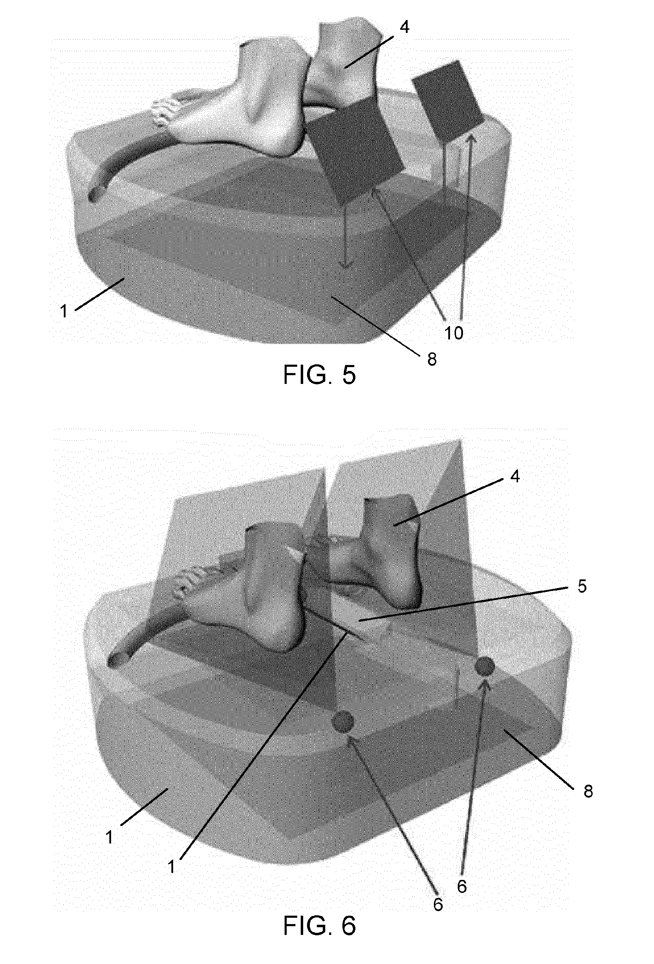

The image of the leg and the heel can be recorded in two ways. In a first variant one or two mirror(s) 10 is (are) placed behind the heel, inclined at an angle of 45° above the examination plate 2 of the podoscope 1. In this case, the image of the leg and the heel is projected to the mirror 8 placed at the bottom of the podoscope 1, and the mirror 8 reflects the image to the mentioned intermediate camera 9. Figure 5 shows a schematic view of this possible arrangement.

Figure 6 outlines a second variant where, instead of using reflecting mirror(s) 10 protruding from podoscope 1, two rear cameras 6 are installed in the podoscope 1, said cameras 6 being able to record the heels and the leg at an oblique angle. Cameras 6 can "look out" from podoscope 1 through the examination plate 2 made of glass and see the rear surface of the foot 4 placed on the examination plate 2 up to the knee of the examined person. This distorted image may and can be corrected by a known computer program. The relevant angle **α** of axes **a** and **b** of the leg and heel can be easily determined with the help of a proper known and suitable graphical utility.

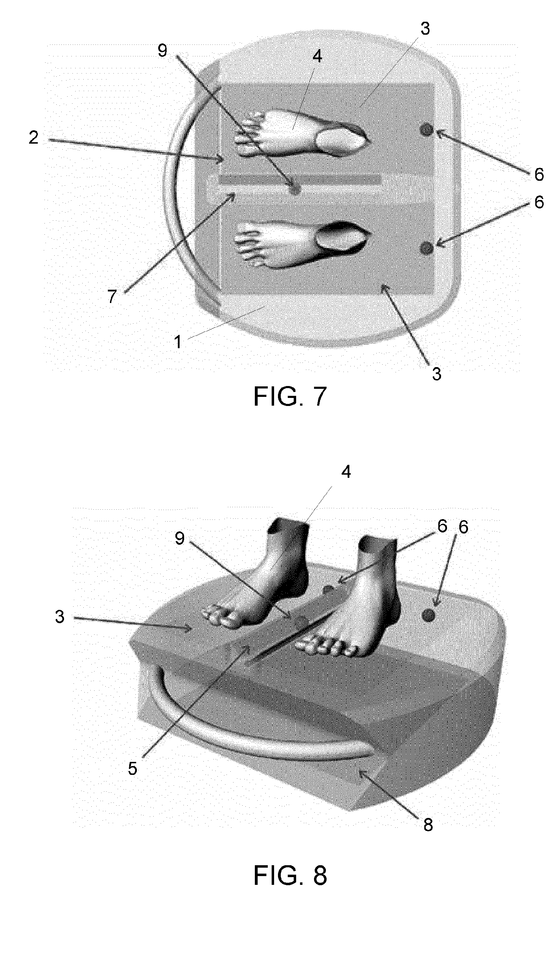

The arrangement presented above is shown in plan view in Figure 7 and in perspective view in Figure 8, and the position of the mentioned specific optical elements and image-recording devices relative to feet 4 is easily discernible in the figures.

The cameras and mirrors of the proposed structural arrangement make it possible to see concurrently on the computer display screen
- the feet 4 of the examined person from behind (leg and heel), (talocrural angle);
- the inner surfaces of both feet 4 (longitudinal arch); and
- the pattern of pressure points of the two soles.

The pressure point pattern is recorded and the arrangement of any necessary transversal arch lifting and other supplementary modules can be realized as disclosed e.g. in HU 222294 B1. The examination of the pressure point pattern assists with the determination of the exact place of the correction modules needed.

In the disclosed embodiment the mirrors of the pair of mirrors 7 as well as mirrors 8 and 10 are flat mirrors. Preferably a battery-powered light source may also be placed inside of the proposed structural arrangement, whereby the optical elements project the emitted light onto the examined sole or soles. The examination plate 2 can be associated with a mechanical-strength-enhancing stiffening lining, and the arrangement can be combined with scales serving for measuring the body weight and/or the body fat percentage.

### List of reference signs:

- a: axis
- b: axis
- c: height
- α: angle
- 1: podoscope
- 2: examination plate
- 3: surface
- 4: foot
- 5: rib
- 6, 9: camera
- 7: pair of mirrors
- 8, 10: mirror

## Claims

1. Structural arrangement for an apparatus for the examination of the pressure load of the sole, comprising
- an examination plate (2) made of at least translucent material, for supporting the sole or both soles of the examined person;
- at least one image recording device being in visual connection with the sole or both soles of the examined person;
- at least one optical element inserted in an optical path between the examination plate (2) and at least one image recording device,
***characterized in* that**
further comprising a pair of mirrors (7) arranged in longitudinal direction between soles of the examined person and used as an optical element for projecting an image of an inner ankle region of the feet (4) of the examined person on a further mirror (8) constituting an optical element and arranged under and parallel to the examination plate (2),
the at least one image recording device comprises a camera (9) arranged in a plane between the soles of the examined person and being in optical connection with said mirror (8), and
one of the image recording device and a further optical element is arranged along the longitudinal axis of the soles of the examined person and assigned to the heel region of the examined sole for combining an image of the heel and at least a partial region of a leg of the examined person with an image reflected by the mirror (8) or delivered by the camera (9).

2. The structural arrangement according to claim 1 ***characterized in* that** the examination plate (2) is made of glass.

3. The structural arrangement according to claim 1 ***characterized in* that** the image recording device comprises a camera (6, 9).

4. The structural arrangement according to claim 1 ***characterized in* that** the optical element comprises a mirror (7, 8).

5. The structural arrangement according to any of claims 1 to 4, ***characterized in* that** the examination plate (2) comprises a transparent plate.

6. The structural arrangement according to any of claims 1 to 5, ***characterized in* that** a further mirror (10) is arranged over the examination plate (2) and tilted 45° away from the plane of the examination plate (2), along the longitudinal axis of the soles of the examined person.

7. The structural arrangement according to any of claims 1 to 5, ***characterized in* that** a further camera (6) is arranged under the examination plate (2) along the longitudinal axis of the soles of the examined person.

8. The structural arrangement according to claim 7 ***characterized in* that** said camera (6) is arranged spaced from and behind the heel region of the examined sole and directed to the sole and at least a partial region of a leg of the examined person.

9. The structural arrangement according to claim 4 or 8 ***characterized by*** comprising plane mirrors (7, 8, 10).

10. The structural arrangement according to any of claims 1 to 9, ***characterized by*** comprising at least one internal light source arranged in a manner projecting its emitted light via the optical elements onto one or both examined soles.

11. The structural arrangement according to any of claims 1 to 10, ***characterized in that*** in case of simultaneous examination of both soles it comprises a single further image recording device and further optical elements assigned to the soles and arranged between them, wherein each of the further optical elements is arranged in longitudinal direction between the soles and tilted away from the vertical plane in opposite directions.

12. The structural arrangement according to any of claims 1 to 10, ***characterized in that*** in case of simultaneous examination of both soles it comprises a further image recording device and a further optical element assigned to each of the soles.

13. The structural arrangement according to any of claims 1 to 12, ***characterized by*** comprising a mechanical stiffener inlay associated with the examination plate (2).

14. The structural arrangement according to any of claims 1 to 13, ***characterized in that*** it is built in combination with a means for measuring body weight and/or body fat percentage.
